# EUROPEAN PATENT SPECIFICATION

(11) **EP 0 468 031 B1**
(45) Date of publication and mention of the grant of the patent: **09.04.1997**
(21) Application number: 91904260.6
(22) Date of filing: 06.02.1991
(51) Int. Cl.: C12Q 1/00, G01N 33/00, G01N 30/02, G01N 24/00, G01N 33/543, G01N 30/90

(54) **QUANTITATIVE ANALYSIS OF CHIROINOSITOL AS A DIABETIC CONDITION PREDICTOR**
QUANTITATIVE ANALYSE VON CHIROINOSITOL ALS ANZEIGER EINES DIABETISCHEN ZUSTANDES
ANALYSE QUANTITATIVE DU CHIRO-INOSITOL COMME INDICATEUR D'UN ETAT DIABETIQUE

(30) Priority: 08.02.1990 US 476953
(43) Date of publication of application: 29.01.1992
(73) Proprietor: THE UNIVERSITY OF VIRGINIA PATENTS FOUNDATION, Charlottesville, Virginia 22903 (US)
(72) Inventor: KENNINGTON, Alison, S., Charlottesville, VA 22901 (US); LARNER, Joseph, Charlottesville, VA 22901 (US)
(74) Representative: VOSSIUS & PARTNER
(86) International application number: US9100655
(87) International publication number: WO9112335

(56) References cited:
- WO-A-90/10711
- THE NEW ENGLAND JOURNAL OF MEDICINE, Volume 323. Number 6, issued 09 August 1990, A.S. KENNINGTON, et al., "Low Urinary chiro-Inositol Excretion in Non- Insulin-Dependent Diabetes Mellitus" pages 373-378. See entire document.
- BIOCHEMICAL and BIOPHYSICAL RESEARCH COMMUNICATIONS, Volume 151, Number 3, issued 30 March 1988, J. LARNER et al., Dehydrogenase Phosphatase Contains Galactosamine and D-Chiroinositol", pages 1416-1426.
- BIOCHEMICAL and BIOPHYSICAL RESEARCH COMMUNCATIONS, Volume 146, Number 2, issued 31 July 1987, J.M. MATO et al., "Partial Structure of an Insulin-Sensitive Glycophospholipid", pages 764-770
- METABOLISM, Volume 35, Number 4, Suppl. 1. issued April 1986. J.R. WILLIAMSON et al., "Diabetes-Induced Increases in Vascular Permeability and Changes in Granulation Tissue Levels of Sorbitol, Mvo-Inositol. Chiro-Inositol, and Scyllo-Inositol are Prevented by Sorbitol" pages 41-45.
- Journal of CHROMATOGRAPHY, Volume 277, issued 1983, T. NIWA et al., "Gas Chromatographic-Mass Spectrometric Analysis of Polyols in Urine and Serum of Uremic Patients", pages 25-39.
- JOURNAL OF CHROMATOGRAPHY, Volume 336, issued 1984, T. NIWA et al., "Identification of 6-deoxyallitol and 6-deoxygulitol in Human Urine: Electron Impact Mass Spectra of Eight Isomers of 6-deoxyhexitol", pages 345-350.

## Description

### Technical Field

This invention pertains to a quantitative assay for a marker or predictor of insulin resistance and the associated diabetic condition in mammals, specifically, a quantitative assay which provides for quick screening of individual patients for an indicator of the diabetic state, the absence of chiro-inositol. The assay provides a low-cost, quick method for conducing a preliminary assay for diabetes, or a predilection to the development of diabetes, in mammals, including humans.

### Background Art

Niwa et al., J. of Chromatography 277 (1983), 25 to 39, report on diabetes-induced changes in the levels of various alcohols, for example chiro-inositol, in the tissue of rats, but do not describe such changes in blood or serum or the correlation of such changes with insulin resistance.

Williamson et al., Metabolism 35 (1986), 41 to 45, report on the gas chromatographic-mass spectrometric analysis of polyols in urine and serum of uremic patients. One of the substances investigated is chiro-inositol. However, this paper does not report on the correlation of the concentration of this alcohol in the context of insulin resistance.

As reported in U.S. Patent No. 5,183,764, discoveries concerning the structures of insulin mediators in mammals, and the presence, in at least one of those mediators, of D-chiro-inositol, led, in part, to the determination that this particular sugar alcohol was absent, or substantially absent, in diabetic individuals, particularly insulin-resistant diabetics, who traditionally are difficult to both identify, and treat.

Further research into this phenomenon led to the determination that both D-chiro-inositol and L-chiro-inositol concentrations in diabetic individuals, particularly type II diabetics, are significantly reduced, and that a quantitative assay for the presence of these "markers" can result in the identification (a) of type II diabetics and (b) individuals at risk or predicted to develop the symptoms of type II diabetes.

U.S. Patent No. 5,183,764 as originally filed, discloses one type of sample to be screened for the presence of D-chiro-inositol. This includes the collection of a 24-hour urine sample, accompanied by convenience of frozen storage, if necessary. Although highly desirable, due to its non-invasive nature, low cost, reliability and speed, further improvements on this process could be obtained.

Accordingly, it remains an object of those of ordinary skill in the art to provide a sensitive, quantitative assay for both D and L-chiro-inositol, as a marker, indicator or predictor of insulin-resistance and type II diabetes.

### Disclosure of the Invention

The above objects, and others more fully developed below, are achieved by a quantitative assay for chiro-inositol taken from spot samples of either mammalian urine or serum. Concentrations of chiro-inositol, relative to both normal individuals, and other inositols, appear constant in both types of samples, eliminating the need for a 24-hour urine specimen collection, and providing alternative fluid sources, both for the convenience of the analyst, and for a check on assay results.

One convenient assay system involves the use of conventional gas chromatography/mass spectrophotometry (GC/MS). Other assay methods are known. Chiro-inositol concentrations of 1 micrograms/ml and above in either urine or serum samples is indicative of the normal or control concentrations for chiro-inositols in healthy individuals. A value of 0.3 micrograms - 1 micrograms per ml is suggestive of a mammalian individual at risk for the development of type II diabetes, or may be further indicative of a presymptomatic type II diabetic. Chiro-inositol values below 0.3 micrograms/ml are indicative of type II diabetics. Chiro-inositol concentrations in serum samples are about 10 fold lower than urine. Thus, values in serum of below 0.1 micrograms/ml are indicative of individuals at risk, and values below about 0.03 are indicative of type II diabetes.

It has further been discovered that similar low inositol concentrations can be correlated with type I diabetics demonstrating insulin-resistance, a characteristic of type II diabetics. Thus, sampling of mammalian populations according to the quantitative assay of this invention appears to be indicative of insulin-resistance, exhibited by a limited number of type I diabetics and type II diabetics. Accordingly, the assay may be used both as a confirmatory assay for the presence of type II diabetes in all populations, a confirmation for insulin-resistance in type I diabetics not responding to insulin therapy, as well as a useful tool for family studies and genetic prediction for insulin-resistant individuals, or individuals drawn from families exhibiting insulin-resistance, for generational planning.

### Best Mode for Carrying Out The Invention

In the performance of the assay of this invention, a urine or serum sample must be obtained. These samples may be spot samples, and no specific time of day or fasting period is required in order to perform the assay with accuracy and precision. Additionally, concentrations of chiro-inositol in patients exhibiting below normal concentrations appear to be independent of various sugar-lowering agents, including insulin and related agents. If a urine sample is obtained, the sample may be adsorbed onto a conventional anion/cation exchange resin bed, eluted with distilled deionized water, and further purified, through solid phase extraction. The sample collection is recovered in distilled water, dried and derivatized. An exemplary derivatization material is heptafluorobutyrylimidazole. In an alternative embodiment of the invention, a serum sample may be collected and subjected, e.g., to ultrafiltration and then applied to purification resins, and subsequently derivatized, as with the urine specimen discussed above.

Conventionally, the samples prepared may be easily assayed on a gas chromatography/mass spectrometer (e.g. HP5710A chromatograph combined with a JMS D-300JEOL spectrometer).

Spot samples exhibiting chiro-inositol concentration (D- and L- combined) of about 1 micrograms/ml and above are representative of normal concentrations, exhibited by non-diabetic, non-insulin-resistant individuals. Assays reflecting values below 0.3 micrograms/ml are insulin-resistant. Type I diabetics exhibiting these substantially absent chiro-inositol concentrations should be considered insulin-resistant, when determining proper therapy. The remaining individuals are expected to be type II diabetics.

Values obtained through the assay of this invention of 0.3 micrograms - 1 micrograms/ml are indicative of individuals at risk of development of type II diabetes, or individuals who are presymptomatic type II diabetics. All such readings should be confirmed, of course, by subsequent more involved assays available to those of skill in the art.

Studies on Pima indian populations of Arizona, as well as a patient population in and about the University of Virginia, Charlottesville, Virginia, and diabetic rhesus monkeys, University of Maryland Primate Center, exhibit a virtual absence of chiro-inositol in diabetic individuals. The marked correlation between chiro-inositol concentrations and insulin-resistance, through these blind-blind studies, was particularly dramatic when compared with the concentrations of structurally related myo-inositol concentrations, which are generally elevated in diabetic patients, and were significantly higher in the diabetic subjects studied than non-diabetics.

Muscle biopsy experiments confirm the findings of low or nonexistent concentrations of chiro-inositol in the patients studied exhibiting non-insulin dependent diabetes mellitus (NIDDM). Given the apparent presence of chiro-inositol in insulin mediators and the correlation between the absence of chiro-inositol and insulin-resistance, applicants believe that there may be a biosynthesis pathway lacking in individuals exhibiting NIDDM and related insulin-resistance or insulin insensitivity, which prevents or retards the formation of a mediator essential in some insulin-activated metabolic pathways. Applicants do not wish to be bound by this theory.

The importance of chiro-inositol as a compound linked to insulin-resistance, and thus linked to individuals requiring non-insulin therapy in the treatment of diabetes, provides a convenient method of monitoring the efficacy of chemical therapy prescribed for the diabetic patient. Those diabetics under some type of chemical therapy, including insulin administration, can be monitored routinely through the assay of this invention for chiro-inositol concentrations. A pronounced drop in the chiro-inositol concentrations, or absence of chiro-inositol from the serum, urine or other sample, is suggestive of insulin-resistance. Additionally, if the individual is on an inositol supplement, in order to treat the deficiency, assays of samples taken some hours subsequent to ingestion of the medication should give an accurate reflection of the value of the treatment.

It should be noted that this invention can be practiced through assays other than GC/MS. A wide variety of assays are known to those of ordinary skill in the art for the detection of specific compounds, and the use of any one which is consistent with a derivatized, dried or liquid sample is appropriate herein. Thus, enzyme reduction/oxidation potential measurements, using, e.g., an immobilized enzyme specific for chiro-inositol, the immobilized enzyme being in a fixed relationship to an electrode, may be used. The presence of chiro-inositol will result in the enzyme-catalyzed reaction going forward, altering the electrical environment detected by the electrode. Alternative assays that can be employed may include antibody assays, e.g., ELISA, LISA, agglutination assays and the like. All such assays are within the context of the invention claimed herein.

The above invention has been described with reference to specific examples and materials. It should be clear that these specifics can be varied without departing from the scope of the invention. In particular, alternative resins in the purification format, alternation of the purification sequence, and alternative assay forms, sensitive to chiro-inositol may be employed, without departing from the scope of the invention, as defined by the claims appended hereto.

## Claims

1. A method for diagnosing a mammalian individual for insulin resistance, comprising:
a) collecting an urine or serum sample from said individual;
b) assaying said sample for the presence of chiro-inositol;
c) comparing the concentration of chiro-inositol in the sample with a concentration which is characteristic of insulin resistance, and
d) classifying the individual as insulin resistant if the concentration of chiro-inositol in the sample is less than or equal to the concentration which is characteristic of insulin resistance.

2. The method of claim 1, wherein urinary chiro-inositol concentrations in said sample below 0.3 µg/mB and serum chiro-inositol concentrations below 0.03 µg/ml are indicative of insulin-resistance in said individual.

3. The method of claim 1 or 2, wherein the insulin resistance is associated with a diabetic state.

4. A method for screening mammalian individuals for a predisposition to insulin resistance comprising the steps of:
a) collecting an urine or serum sample from the individual;
b) assaying said sample for the presence of chiro-inositol;
c) comparing the concentration of chiro-inositol in the sample with a concentration which is characteristic of a predisposition to insulin resistance, and
d) classifying the individual as having a predisposition to insulin resistance if the concentration of chiro-inositol in the sample is less than or equal to the concentration which is characteristic of a predisposition to insulin resistance.

5. The method of claim 4, wherein urinary chiro-inositol concentrations in said sample between 0.3 µg/ml and 1.0 µg/ml and serum chiro-inositol concentrations below 0.1 µg/ml are indicative of a predisposition to insulin-resistance in said individual.

6. A method for monitoring the efficacy of insulin therapy administered to a diabetic patient, comprising:
a) collecting urine or serum samples from said diabetic patient,
b) assaying said sample for the presence of chiro-inositol, and
c) comparing the concentration of chiro-inositol in said sample and of samples taken some hours subsequent to ingestion of the medication.

7. The method of any one of claims 1 to 5 wherein said individual is a human.

8. The method of any one of claims 1 to 6 further comprising the step of purifying said sample after step a).

9. The method of claim 8 wherein said sample is purified by anion/cation exchange chromatography and solid phase extraction and derivatized prior to step b).

10. The method of claim 8 wherein the purification of said sample comprises ultrafiltration and derivatization prior to step b).

11. The method of any one of claims 1 to 10 wherein step b) is practiced using an assay selected from the group consisting of gas chromatographic/mass spectrometric analysis, paper chromatography, enzyme specific reduction/oxidation potential analysis, wherein said enzyme is specific for chiro-inositol and an ELISA-, LISA- or agglutination assay employing an antibody specific to chiro-inositol.

## Patentansprüche

1. Verfahren für die Diagnose einer Insulinresistenz bei einem Säugerindividuum, umfassend:
a) Sammeln einer Urin- oder Serumprobe von dem Individuum;
b) Analysieren der Probe auf das Vorhandensein von Chiro-Inosit;
c) Vergleichen der Konzentration von Chiro-Inosit in der Probe mit einer Konzentration, die charakteristisch für Insulinresistenz ist, und
d) Klassifizieren des Individuums als insulinresistent, wenn die Konzentration von Chiro-Inosit in der Probe geringer als oder gleich der Konzentration ist, die charakteristisch für Insulinresistenz ist.

2. Verfahren nach Anspruch 1, worin Urin-Chiro-Inosit-Konzentrationen in der Probe von unter 0,3 µg/ml und die Serum-Chiro-Inosit-Konzentrationen von unter 0,03 µg/ml auf eine Insulinresistenz in dem Individuum hinweisen.

3. Verfahren nach Anspruch 1 oder 2, worin die Insulinresistenz begleitend mit einem diabetischen Zustand auftritt.

4. Verfahren für das Durchmustern von Säugetierindividuen auf eine Veranlagung für Insulinresistenz, umfassend die Schritte:
a) Sammeln einer Urin- oder Serumprobe von dem Individuum;
b) Analysieren der Probe auf das Vorhandensein von Chiro-Inosit;
c) Vergleichen der Konzentration von Chiro-Inosit in der Probe mit einer Konzentration, die charakteristisch für eine Veranlagung für Insulinresistenz ist, und
d) Klassifizieren des Individuums als Individuum mit einer Veranlagung für Insulinresistenz, wenn die Konzentration von Chiro-Inosit in der Probe geringer als oder gleich der Konzentration ist, die charakteristisch für eine Veranlagung für Insulinresistenz ist.

5. Verfahren nach Anspruch 4, worin Urin-Chiro-Inosit-Konzentrationen in der Probe zwischen 0,3 µg/ml und 1,0 µg/ml und Serum-Chiro-Inosit-Konzentrationen von unter 0,1 µg/ml auf die Veranlagung für Insulinresistenz in dem Individuum hinweisen.

6. Verfahren für die Kontrolle der Wirksamkeit einer Insulintherapie, die an einem diabetischen Patienten durchgeführt wird, umfassend:
a) Sammeln der Urin- oder Serumproben von dem diabetischen Patienten,
b) Analysieren der Probe auf das Vorhandensein von Chiro-Inosit, und
c) Vergleichen der Konzentration von Chiro-Inosit in der Probe und von Proben, die einige Stunden nach der Einnahme des Medikaments genommen wurden.

7. Verfahren nach einem der Ansprüche 1 bis 5, worin das Individuum ein Mensch ist.

8. Verfahren nach einem der Ansprüche 1 bis 6, weiter umfassend den Schritt der Reinigung der Probe nach Schritt a).

9. Verfahren nach Anspruch 8, worin die Probe durch Anionen/Kationenaustauschchromatographie und Festphasenextraktion gereinigt wird und vor Schritt b) derivatisiert wird.

10. Verfahren nach Anspruch 8, worin die Reinigung der Probe eine Ultrafiltration und Derivatisierung vor Schritt b) umfaßt.

11. Verfahren nach einem der Ansprüche 1 bis 10, worin Schritt b) unter Verwendung eines Tests erfolgt, der ausgewählt ist aus Gaschromatographie/Massenspektrometrieanalyse, Papierchromatographie, enzymspezifische Reduktion/Oxidationspotential-Analyse, worin das Enzym spezifisch für Chiro-Inosit ist, und einem ELISA-, LISA- oder Agglutinationstest unter Verwendung eines Antikörpers, der spezifisch für Chiro-Inosit ist.

## Revendications

1. Méthode de diagnostic de l'insulinorésistance chez un individu mammifère, comprenant:
a) le prélèvement d'un échantillon d'urine ou de sérum dudit individu;
b) le dosage dudit échantillon pour la présence de chiro-inositol;
c) la comparaison de la concentration de chiro-inositol dans l'échantillon avec une concentration caractéristique de l'insulinorésistance, et
d) le classement de l'individu comme étant atteint de l'insulinorésistance si la concentration de chiro-inositol dans l'échantillon est inférieure ou égale à la concentration caractéristique de l'insulinorésistance.

2. Méthode selon la revendication 1, caractérisée en ce que des concentrations urinaires de chiro-inositol dans ledit échantillon inférieures à 0,3 µg/ml et des concentrations sériques de chiro-inositol inférieures à 0,03 µg/ml sont indicatrices d'une insulinorésistance chez ledit individu.

3. Méthode selon la revendication 1 ou 2 caractérisée en ce que l'insulinorésistance est associée à un état diabétique.

4. Méthode de dépistage de la prédisposition à l'insulinorésistance des individus mammifères comprenant les étapes de:
a) prélèvement d'un échantillon d'urine ou de sérum de l'individu;
b) dosage dudit échantillon pour la présence de chiro-inositol;
c) comparaison de la concentration de chiro-inositol dans l'échantillon avec une concentration caractéristique d'une prédisposition à l'insulinorésistance, et
d) classement de l'individu comme ayant une prédisposition à l'insulinorésistance si la concentration de chiro-inositol dans l'échantillon est inférieure ou égale à la concentration caractéristique d'une prédisposition à l'insulinorésistance.

5. Méthode selon la revendication 4, caractérisée en ce que des concentrations urinaires de chiro-inositol dans ledit échantillon comprises entre 0,3 µg/ml et 1,0 µg/ml et des concentrations sériques de chiro-inositol inférieures à 0,1 µg/ml sont indicatrices d'une prédisposition à l'insulinorésistance chez ledit individu.

6. Méthode de suivi de l'efficacité d'une insulinothérapie administrée à un patient diabétique comprenant:
a) le prélèvement d'échantillons d'urine ou de sérum dudit patient diabétique,
b) le dosage dudit échantillon pour la présence de chiro-inositol, et
c) la comparaison de la concentration de chiro-inositol dans ledit échantillon et d'échantillons prélevés quelques heures suivant l'ingestion de la médication.

7. Méthode selon l'une quelconque des revendications 1 à 5, caractérisée en ce que ledit individu est un être humain.

8. Méthode selon l'une quelconque des revendications 1 à 6, comprenant en outre l'étape de purification dudit échantillon après l'étape a).

9. Méthode selon la revendication 8, caractérisée en ce que ledit échantillon est purifié par chromatographie d'échange d'anions/cations et par extraction en phase solide, et dérivatisé préalablement à l'étape b).

10. Méthode selon la revendication 8, caractérisée en ce que la purification dudit échantillon comprend l'ultrafiltration et la dérivatisation préalablement à l'étape b).

11. Méthode selon l'une quelconque des revendications 1 à 10, caractérisée en ce que l'étape b) est mise en oeuvre à l'aide d'un dosage choisi dans le groupe constitué d'analyse chromatographique en phase gazeuse/spectométrique de masse, de chromatographie sur papier, d'analyse du potentiel de réduction/d'oxydation spécifique d'enzyme, caractérisée en ce que ladite enzyme est spécifique du chiro-inositol et d'un dosage ELISA, LISA ou d'agglutination mettant en oeuvre un anticorps spécifique du chiro-inositol.
